# DEMANDE DE BREVET EUROPEEN

(11) **EP 0 884 035 A1**
(43) Date de publication de la demande: **16.12.1998**
(21) Numéro de dépôt: 98390007.7
(22) Date de dépôt: 05.06.1998
(51) Int. Cl.: A61F 5/01

(54) **Orthèse de genou**

(30) Priorité: 09.06.1997 FR 9707127
(71) Demandeur: Organisation pour les Echanges Commerciaux SA, 64600 Anglet (FR)
(72) Inventeur: Darmana, Robert, 31100 Toulouse (FR); Roux, Jean-Louis, 40530 Labenne (FR); Avril, Alain, 64990 Mouguerre (FR)
(74) Mandataire: Cabinet BARRE LAFORGUE & associés

(57) **Abrégé**

L'invention concerne une orthèse de genou, comportant une armature de fémur (2) et une armature de tibia (3), reliées par une articulation (14) propre à permettre un pivotement relatif du fémur par rapport au tibia. L'articulation (14) comprend des structures de contact et de guidage adaptées pour engendrer d'une part, au cours d'une flexion d'angle (a) à partir de la position d'extension complète, une translation vers l'arrière (T) de l'armature de fémur par rapport à l'armature de tibia, se combinant à une rotation d'une armature par rapport à l'autre, d'autre part, après la phase de flexion d'angle (α), une rotation pure des armatures autour d'un doigt arrêté à l'extrémité d'une lumière oblongue. Les structures ont des profils adaptés pour que l'angle de flexion (α) soit de préférence compris entre 15° et 25°.

## Description

La présente invention est relative à une orthèse de genou destinée à être placée autour du genou d'un patient pour permettre à celui-ci de pallier une défaillance fonctionnelle, suite à une opération chirurgicale, un traumatisme ou à une pathologie, en autorisant dans des conditions de maintien satisfaisantes la flexion et l'extension du genou, avec une cinématique proche de celle d'un genou physiologique.

On sait que le genou humain est une articulation particulièrement sophistiquée en raison du nombre d'éléments qui la composent, et des fonctions relatives de ces éléments qui assurent une cinématique complexe. Schématiquement résumé à la description de ses éléments essentiels, un genou comporte des condyles fémoraux, situés à l'extrémité distale du fémur, deux plateaux tibiaux situés sur la partie supérieure du tibia sur lesquels les condyles fémoraux peuvent se déplacer en translation et en rotation, en modifiant la position relative des axes du fémur et du tibia, des ligaments divers, croisés, postérieurs et antérieurs et latéraux, qui relient les diverses parties du genou pour assurer la transmission des efforts et les mouvements relatifs, et enfin des ménisques, sorte de lames cartilagineuses en forme de croissants, situées entre les parties osseuses sur les bords latéraux des plateaux tibiaux pour compenser leurs débattements relatifs.

Sans entrer dans des détails inutiles pour la description et la compréhension de la présente invention, on peut seulement rappeler que l'agencement des diverses parties d'un genou engendre, au cours de la flexion, un mouvement complexe qui combine un déplacement en translation relative du fémur et des diverses parties qui lui sont liées par rapport au tibia sur la surface des plateaux tibiaux, et une rotation relative du fémur par rapport au tibia autour de plusieurs axes instantanés de rotation transversaux passant par les condyles fémoraux.

On connaît déjà dans la technique de nombreux dispositifs d'articulation adaptés pour être montés au droit du genou d'un patient afin d'autoriser un pivotement du fémur par rapport au tibia.

Une première famille d'orthèse du genou assure un guidage partiel du fémur par rapport au tibia mais laisse un degré de liberté dans le sens antéro-postérieur, et ce sont les ligaments du patient qui contrôlent la position antéro-postérieure précise du fémur (brevet GB-2 161 388, WO 88.04542). Ce type d'orthèse n'est applicable que si les ligaments du patient sont sains et peut au contraire être préjudiciable dans le cas d'une articulation où les ligaments sont lésés car le travail demandé à ceux-ci peut participer à accroître ces lésions.

L'orthèse visée par l'invention fait partie d'une autre famille assurant un guidage complet du fémur par rapport au tibia de façon à définir totalement la position de l'un par rapport à l'autre au cours de la flexion sans participation des ligaments naturels. La difficulté rencontrée dans ce type d'orthèse est de reproduire le plus fidèlement possible la cinématique naturelle du genou, afin d'éviter des contraintes et surcharges sur des parties lésées en vue de permettre une réhabilitation satisfaisante.

Un premier type d'orthèse de cette famille est constitué par une articulation monocentrique qui engendre une rotation pure autour d'un axe fixe (par exemples : US-Re 34318, EU-0466538, EU-0633007, US-5 031 606, EU-0677282, US-4 088 130). Dans ce cas, des interférences préjudiciables apparaissent entre l'orthèse artificielle et les éléments (lésés ou non) de l'articulation physiologique, qui contrarient fortement la réhabilitation.

Dans un autre type d'orthèse dite polycentrique, on s'est attaché à combiner mouvement de translation et mouvement de rotation afin de tenter d'éviter les contraintes et surcharges sur les organes physiologiques. Toutes les orthèses polycentriques, existant à l'heure actuelle, proposent des articulations qui, au cours de la flexion, produisent un mouvement combinant une translation vers l'avant du fémur par rapport au tibia et une rotation relative de ces organes autour de plusieurs centres instantanés de rotation. Dans certaines orthèses, la translation vers l'avant du fémur est produite sur toute l'amplitude de la flexion (US-5 632 725, WO 95/03013) ; dans d'autres, la translation vers l'avant du fémur n'est engendrée qu'au début de la flexion (US-4 603 690, US-5 286 250). Toutefois, toutes ces prothèses perturbent gravement le mouvement physiologique du genou et, par voie de conséquence, engendrent nécessairement les contraintes et surcharges précitées.

La présente invention se propose de fournir une orthèse perfectionnée, réduisant au cours de la flexion les contraintes et surcharges subies par les organes naturels, par comparaison aux orthèses connues.

A cet effet, l'orthèse visée par l'invention est une orthèse de type polycentrique, comportant une armature de fémur, des moyens de fixation de cette armature de fémur sur la cuisse d'un patient, une armature de tibia, des moyens de fixation de cette armature de tibia sur la jambe du patient au-dessous du genou, des moyens d'articulation de l'armature de fémur et de l'armature de tibia comprenant au moins deux pièces d'articulation, dites pièce d'articulation fémorale et pièce d'articulation tibiale, respectivement solidaires de l'armature de fémur et de l'armature de tibia et pourvues d'une came d'appui et d'une rampe de guidage agencées pour coopérer en vue d'autoriser et guider le pivotement relatif d'une armature par rapport à l'autre entre une position d'extension complète et une position de flexion complète, des moyens d'arrêt adaptés pour définir la position d'extension complète et interdire une hyperextension, et des moyens d'arrêt adaptés pour définir la position de flexion complète et limiter le pivotement à cette position. Conformément à la présente invention, ladite orthèse se caractérise en ce que ses moyens d'articulation comprennent :
- de premières surfaces de contact conjuguées, portées par la came d'appui et par la rampe de guidage et agencées pour prendre appui et rouler l'une sur l'autre et engendrer, au cours d'une flexion d'angle (α) à partir de la position d'extension complète, une translation vers l'arrière (T) de l'armature de fémur par rapport à l'armature de tibia, se combinant à une rotation (R1) d'une armature par rapport à l'autre,
- un doigt et une lumière oblongue portés par la pièce d'articulation fémorale et par la pièce d'articulation tibiale, ledit doigt étant logé dans ladite lumière oblongue, laquelle est agencée pour autoriser le déplacement vers l'arrière dudit doigt au cours de la flexion d'angle (α), et pour arrêter le doigt en position arrière à la fin de ladite flexion d'angle (α) en vue d'interrompre le mouvement de translation vers l'arrière (T) de l'armature de fémur par rapport à l'armature de tibia après le pivotement d'angle (α) à partir de la position d'extension complète,
- de secondes surfaces de contact conjuguées portées par la came d'appui et par la rampe de guidage et agencées pour glisser l'une sur l'autre après la phase de flexion d'angle (α) de façon à engendrer une rotation pure (R2) des armatures autour du doigt arrêté dans sa lumière oblongue.

Pour simplifier la description, on considère que l'armature de tibia est fixe et que c'est l'armature de fémur qui subit la flexion par rapport à la première.

L'orthèse conforme à l'invention a été conçue et mise au point à partir d'une analyse de la cinématique physiologique du genou et d'une modélisation de cette cinématique pour en fournir une excellente approximation et permettre de la reproduire à l'aide de moyens simples. Ainsi, on a pu s'apercevoir que les orthèses polycentriques connues à ce jour réalisaient la phase de translation (fémur se déplaçant vers l'avant par rapport au tibia) à l'inverse de ce qui se passe dans la physiologie. Dans cette dernière en effet, le fémur subit une translation vers l'arrière par rapport au tibia, et ce pendant toute la flexion, mais d'amplitude décroissante au cours de la flexion pour s'annuler à la fin du mouvement de flexion. Ainsi, il n'est pas surprenant que les orthèses polycentriques connues qui opèrent une translation opposée à la translation physiologique engendrent des contraintes notamment ligamentaires provenant d'un décalage antéro-postérieur du fémur par rapport au tibia. Toutefois, produire une translation continue d'amplitude décroissante du fémur par rapport au tibia conduirait à des systèmes mécaniques très complexes et, en fait, non nécessaires pour obtenir une suppression pratique des contraintes, compte tenu de la relative souplesse permise par les moyens de fixation des armatures sur la cuisse et sur la jambe du patient. L'orthèse de l'invention engendre un mouvement simplifié qui correspond à une modélisation très proche du mouvement physiologique, mouvement simplifié consistant à substituer à la translation arrière continue décroissante du fémur sur toute la flexion, une translation arrière linéaire dans une phase (α) de début de flexion, et à réaliser ensuite une rotation pure (du type de celle des orthèses monocentriques) après cette phase de début. On obtient ainsi, à la fois une grande simplicité des moyens mis en oeuvre et une cinématique reproduisant de façon satisfaisante la cinématique physiologique (compte tenu des jeux de fixation sur la cuisse et la jambe du patient).

Selon un mode de réalisation préférentiel, la came d'appui et la rampe de guidage ainsi que le doigt et la lumière oblongue sont adaptés pour conditionner une translation vers l'arrière de l'armature de fémur par rapport à l'armature de tibia sur un angle de flexion (α) sensiblement compris entre 15° et 25°. On a constaté qu'un tel angle (α) conduisait à une approximation optimale de la cinématique physiologique.

Par ailleurs, l'orthèse comprend avantageusement, de façon connue en soi (US-Re 34318), des moyens réglables de limitation de la flexion et de l'extension, comportant une pluralité de logements répartis sur la pièce d'articulation tibiale, un bossage solidaire de la pièce d'articulation fémorale, et au moins un téton logé dans un desdits logements en vue de servir de butée au bossage dans la position limite désirée de flexion ou d'extension. Ces moyens permettent au chirurgien ou au kinésithérapeute, de limiter la flexion ou l'extension sur une plage angulaire donnée en fonction de la thérapie souhaitée. Cette plage angulaire peut ainsi être modifiée au cours de la réhabilitation, en particulier pour l'élargir progressivement. Dans un mode de réalisation, l'écartement angulaire est sensiblement égal d'un logement au suivant et correspond sensiblement à un angle de 10°, le nombre des logements étant choisi de manière à autoriser un mouvement en flexion d'amplitude maximale comprise entre environ 0° à 110°, et en extension de 0° à 90° environ.

Selon un mode de réalisation préféré, l'orthèse comprend deux pièces d'articulation fémorale et deux pièces d'articulation tibiale, destinées à coopérer par couples de part et d'autre du genou, une coque de protection de rotule étant fixée sur les deux pièces d'articulation tibiale. Une telle orthèse utilisée en pathologie assure en complément une protection de la rotule. Le cas échéant, elle peut être utilisée sur des sujets sains, fragiles ou ayant pour seule pathologie une lésion rotulienne, en vue d'assurer une protection de la rotule et un soulagement des ligaments.

D'autres caractéristiques d'une orthèse de genou conforme à l'invention ressortiront de la description qui suit d'un exemple de réalisation, donné à titre non limitatif, en référence aux dessins annexés sur lesquels :
- la figure 1 est une vue schématique en perspective de l'orthèse considérée,
- la figure 2 est une vue de côté et en coupe longitudinale de celle-ci,
- la figure 3 est une vue éclatée des divers éléments de l'articulation de l'orthèse,
- la figure 4 est une vue en coupe, également éclatée, des éléments illustrés sur la figure 3,
- la figure 5 est une vue en coupe et en détail de l'extrémité de la pièce 2,
- les figures 6, 7, 8, 9, 10 et 11 sont des schémas illustrant le mouvement de flexion de l'orthèse depuis une position d'extension complète jusqu'à une position de flexion complète,
- enfin la figure 12 montre une orthèse de type précité, pourvue d'une coque de protection de rotule.

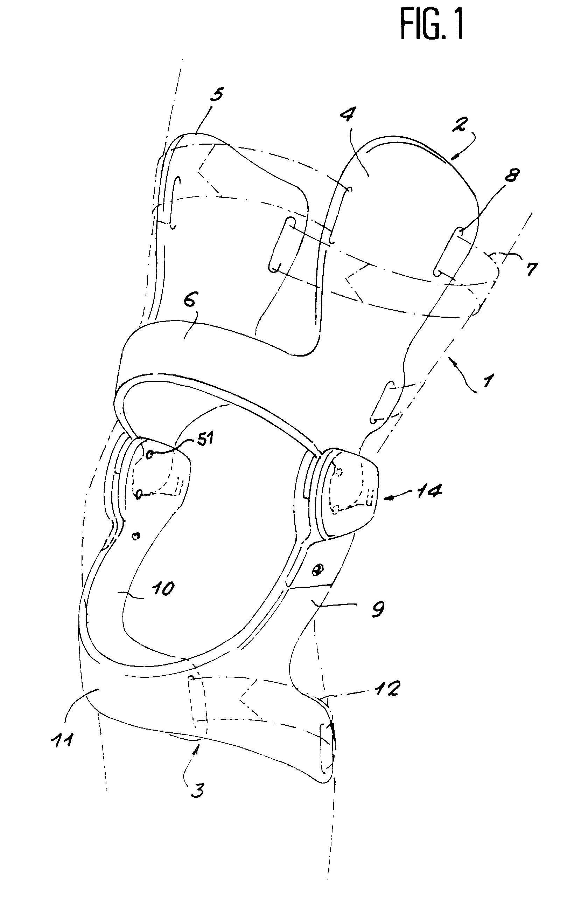

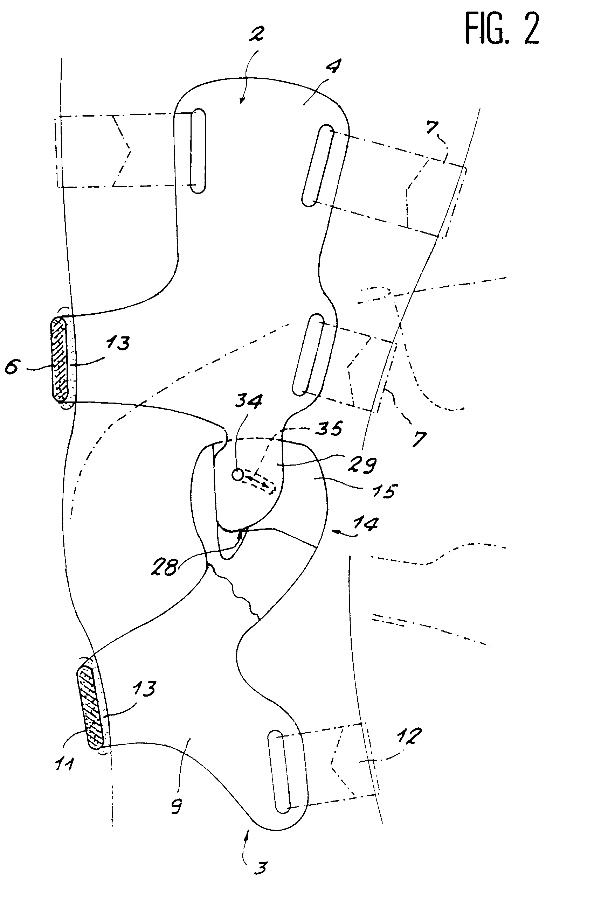

Sur les figures 1 et 2, l'orthèse 1 comporte une armature de fémur 2 et une armature de tibia 3, respectivement prévues pour être solidarisées au fémur et au tibia d'un patient.

L'armature de fémur 2 se présente de préférence sous la forme de deux attelles latérales rigides 4 et 5, réunies sur le devant de la cuisse au-dessus du genou par un arceau antérieur rigide 6, l'ensemble étant immobilisé en position sur le patient au moyen de deux sangles 7, passant dans des oeillets 8 ménagés dans les attelles et se fermant derrière le membre. L'arceau antérieur 6 est disposé à la base de l'armature de fémur 2 de façon à procurer un appui au bas de la cuisse du patient à proximité immédiate du genou.

L'armature de tibia 3 présente sensiblement la même structure, avec deux attelles latérales 9 et 10, un arceau antérieur rigide de liaison 11 situé sous le genou, et une sangle de fixation 12. L'arceau antérieur 11 est disposé en position à peu près médiane par rapport à l'armature de tibia 3 de façon à procurer un appui en partie haute du tibia en préservant, au-dessus dudit arceau, la place du genou.

Les sangles 7 et 12 peuvent avantageusement comporter des moyens de réglage de leur longueur pour permettre leur serrage plus ou moins ajusté respectivement sur la cuisse et la jambe, et sont munies d'éléments d'accrochage de leurs extrémités qui peuvent être d'un type quelconque, du gendre bande "Velcro" ou autre.

Les arceaux 6 et 11, comme les attelles 4 et 5, 9 et 10, sont de préférence revêtus à l'intérieur d'une couche de mousse élastique 13 ou analogue pour améliorer le confort du patient lorsque le dispositif est mis en place. L'agencement en hauteur des arceaux 6 et 11 précédemment défini confère un excellent maintien antéro-postérieur de l'articulation physiologique en raison de la rigidité desdits arceaux et de la proximité des appuis qu'ils procurent de part et d'autre du genou.

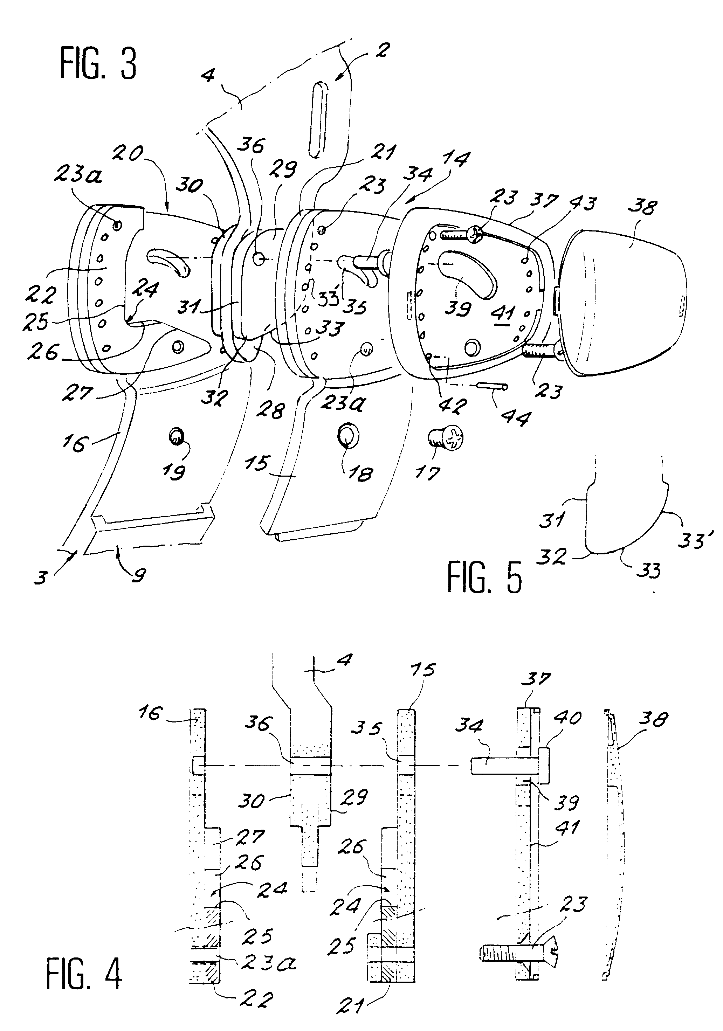

Les deux armatures 2 et 3 sont réunies au niveau du genou qu'elles entourent, par une articulation 14 dont le détail de la réalisation est plus particulièrement illustré sur les figures 3 et 4, cette articulation permettant, conformément à l'invention, d'autoriser en cours de flexion, un double mouvement de translation vers l'arrière de l'armature de fémur et de rotation relative desdites armatures, l'amplitude de ce dernier mouvement étant réglable en fonction du programme de rééducation du patient.

Si l'on se réfère plus particulièrement à ces figures 3 et 4, on retrouve sur ces dernières les attelles 4 et 9 des armatures de fémur 2 et de tibia 3, les autres attelles 5 et 10 situées de l'autre côté de l'orthèse n'étant pas représentées pour ne pas surcharger inutilement le dessin.

L'attelle 9 forme à son extrémité supérieure une pièce d'articulation tibiale comprenant deux plaques parallèles espacées, respectivement 15 et 16, propres à être solidarisées l'une avec l'autre dans le prolongement supérieur de l'armature de tibia 3 par un rivet 17 ou analogue, traversant des orifices 18 et 19 prévus dans les plaques 15 et 16, ces dernières étant aménagées à leur partie supérieure de manière à délimiter entre elles une fente 20 ouverte vers le haut, dans laquelle vient s'engager la partie inférieure de l'attelle 4 comme précisé ci-après.

Les plaques parallèles 15 et 16 de l'attelle 9 sont associées, au droit de la fente 20, à deux guides plats respectivement 21 et 22 formant des rampes de guidage, ces guides étant fixés aux plaques par des rivets 23 engagés dans des perçages 23a correspondants. Ces guides, exactement symétriques l'un de l'autre par rapport au plan médian de la fente, présentent chacun une encoche ouverte 24, seule celle illustrée sur le guide 22 solidaire de la plaque 16 apparaissant sur la figure 3, les deux encoches étant en revanche visibles sur la vue en coupe de la figure 4.

L'encoche 24 comporte un arrondi concave de faible rayon de courbure, prolongé d'un côté et de l'autre d'une part, d'une face plane 25 sensiblement verticale (dans la position tibia dressé à la verticale), d'autre part, d'une face plane 26 qui s'étend dans une direction sensiblement perpendiculaire à la face plane 25, cette face se terminant elle-même par une face inclinée 27.

L'attelle 4 de l'armature de fémur 2 forme à son extrémité inférieure une pièce d'articulation fémorale qui pénètre à l'intérieur de la fente 20 ménagée entre les plaques 15 et 16, et comporte de part et d'autre deux plaques identiques latérales 29 et 30, symétriques l'une de l'autre par rapport à la partie inférieure de l'attelle 4. Ces plaques 29 et 30 fixées sur cette partie inférieure forment une came d'appui comprenant un profil arrondi convexe 32 de faible rayon de courbure, prolongé vers l'arrière par un profil courbe 33 et, vers le haut, d'une face frontale plane 31 sensiblement verticale et donc parallèle à la face 25 de l'encoche ouverte 24 prévue dans les guides 21 et 22.

La came d'appui comprend en outre un profil courbe 33' situé dans le prolongement du profil courbe 33, ce profil courbe étant adapté pour venir glisser sur la face inclinée 27 de la rampe de guidage, une fois accomplie la phase de début de flexion (α).

Ainsi, le profil arrondi 32 de la came d'appui, prolongé par le profil courbe 33, d'une part, et l'encoche ouverte 24 de la rampe de guidage, prolongée par les faces planes 25 et 26, forment de premières surfaces de contact conjuguées, appelées à prendre appui et rouler l'une sur l'autre en vue d'engendrer, au début du mouvement de flexion, une translation vers l'arrière de l'armature de fémur par rapport à l'armature de tibia, se combinant à une rotation d'une armature par rapport à l'autre. Pendant ce début de flexion d'angle α, le profil arrondi 32 vient se loger dans l'encoche ouverte 24 pour servir de guide de roulement et vient en appui sur la face 25 pour servir de butée avant et assurer la translation vers l'arrière, cependant que le profil courbe 33 vient rouler sur la face plane 26. Ces surfaces de contact sont adaptées pour produire une première flexion d'un angle avantageusement voisin de 20°.

En outre, le profil courbe 33' de la came d'appui, d'une part, et la face inclinée 27 de la rampe de guidage, d'autre part, forment de secondes surfaces de contact conjuguées, appelées à prendre appui et glisser l'une sur l'autre en vue d'engendrer, après la phase de flexion d'angle α, une rotation pure de l'armature de fémur par rapport à l'armature de tibia.

Un doigt 34 est monté dans un trou 36 de la pièce d'articulation fémorale (plaques 29, 30, extrémité de l'attelle 4) pour traverser de part en part celle-ci et venir se loger dans des lumières oblongues 35 ménagées en correspondance dans les plaques espacées 15, 16 de la pièce d'articulation tibiale. Ce doigt 34 et la lumière oblongue 35 conjuguée sont agencés de sorte que le doigt 34 soit libre de se déplacer vers l'arrière au cours de la flexion d'angle a (avantageusement 20°) et vienne en butée contre l'extrémité de la lumière à la fin de cette flexion d'angle α en vue d'interrompre le mouvement de translation vers l'arrière et de produire un mouvement de rotation pure pendant la dernière phase de flexion.

La plaque 15 de la pièce d'articulation tibiale, située vers l'extérieur de l'articulation, est par ailleurs associée à un boîtier 37 muni d'un couvercle 38, permettant de fermer ou d'ouvrir à volonté ce boîtier. Celui-ci comporte une ouverture interne 39 en regard de la lumière oblongue 35 des plaques 15 et 16; le doigt 34 comporte une tête épaulée 40 disposée de l'autre côté du fond 41 dudit boîtier 37, de manière à rendre ce doigt prisonnier du boîtier sans empêcher son déplacement dans la lumière oblongue.

Comme on le voit sur la figure 3, le fond 41 du boîtier comporte une pluralité de logements 42 ou 43, selon qu'ils sont situés vers l'avant ou vers l'arrière de l'articulation. Ces logements se prolongent dans les plaques 15 et 16, et les guides plats 21 et 22 de la pièce d'articulation tibiale ; ces logements sont prévus pour recevoir un téton tel que schématiquement représenté en 44, le rôle de ce téton étant de former butée d'arrêt pour limiter les mouvements de flexion et d'extension de l'articulation par arrêt d'un bossage 28 solidaire de la pièce d'articulation fémorale (constitué en pratique par une extension d'extrémité de l'attelle 4).

Le fonctionnement de l'orthèse décrite ci-dessus est le suivant.

Le patient dont la cuisse et la jambe sont équipées des deux armatures 2 et 3 convenablement immobilisées par leurs sangles 7 et 12, peut, grâce à l'articulation proposée, faire subir à son genou des mouvements aussi bien de flexion que d'extension, ces mouvements étant contrôlés (et éventuellement limités) par l'orthèse qui reproduit selon un modèle fidèle la cinématique du genou physiologique.

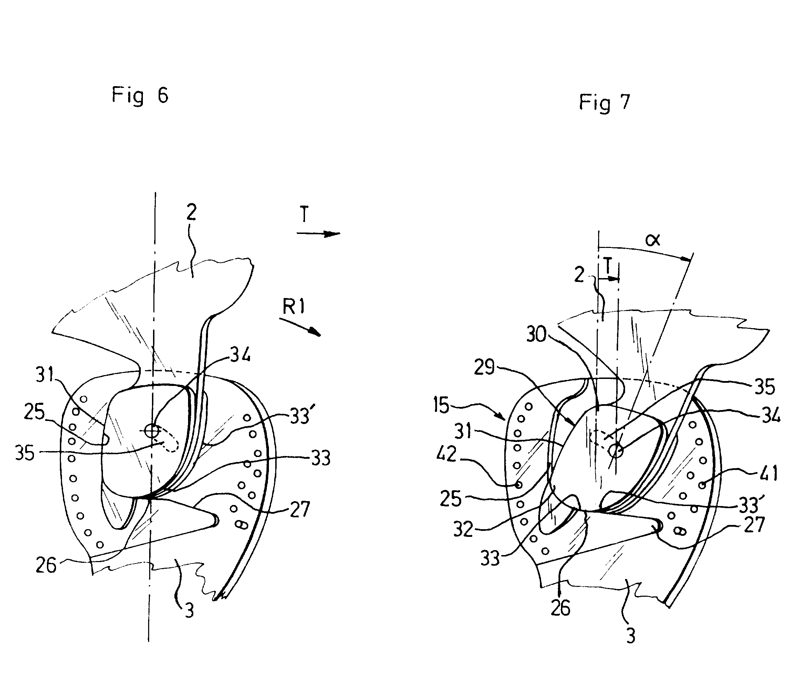

En extension complète (schéma de la figure 6), la face 31 de la came d'appui de l'armature de fémur s'appuie sur la face plane 25 de la rampe de guidage de l'armature de tibia. Cet appui, de même que la présence du doigt 34, empêche un basculement vers l'avant du fémur par rapport au tibia et reproduit ainsi le blocage des ligaments croisés du genou, empêchant dans cette position une hyper extension.

Lorsque le patient fait fléchir l'articulation jusqu'à l'angle a de 20° en modifiant la position angulaire relative du fémur par rapport au tibia, se produit une rotation relative R1 de l'armature de fémur 2 par rapport à l'armature de tibia 3, cette rotation étant combinée avec une translation T vers l'arrière (figure 7). En effet, lors du début de la rotation du fémur, la face 31 quitte l'appui avec la face 25, le centre de pivotement étant défini par l'arrondi 32 de faible rayon de courbure. Du fait du roulement, la zone d'appui se déplace pendant la flexion du fémur pour venir se situer sur la face 26 et le profil courbe 33. L'arrondi 32 vient en butée sur la partie basse de la face 25 et pousse ainsi vers l'arrière l'armature fémorale 2. Le mouvement de translation arrière T combiné avec la rotation R1 reproduit la cinématique du genou physiologique des 20 premiers degrés de flexion. Les amplitudes de rotation et de translation sont obtenues par le dimensionnement correct des plaques 29 et 30 et de l'encoche 24 et en particulier par le profil et le rayon des surfaces de contact 31, 32, 33, 25, 26.

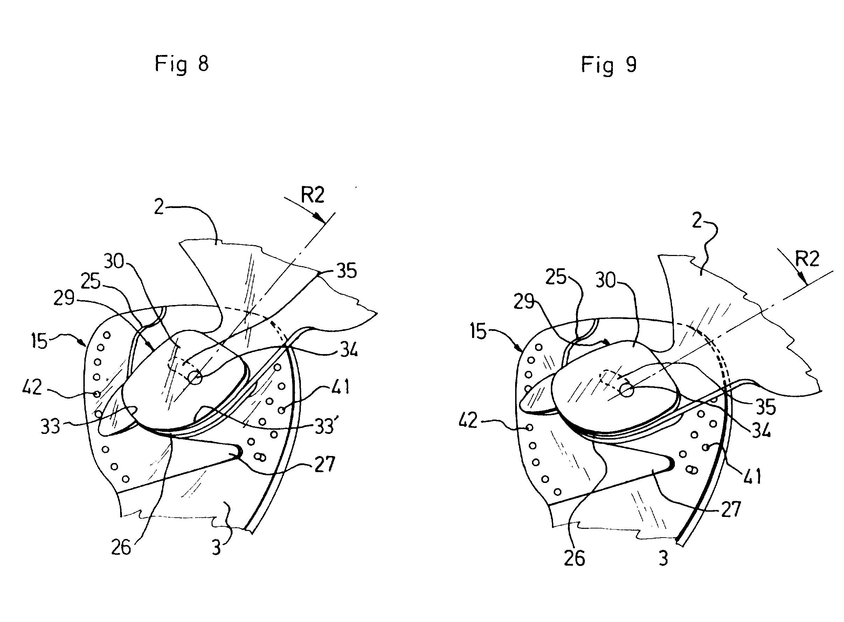

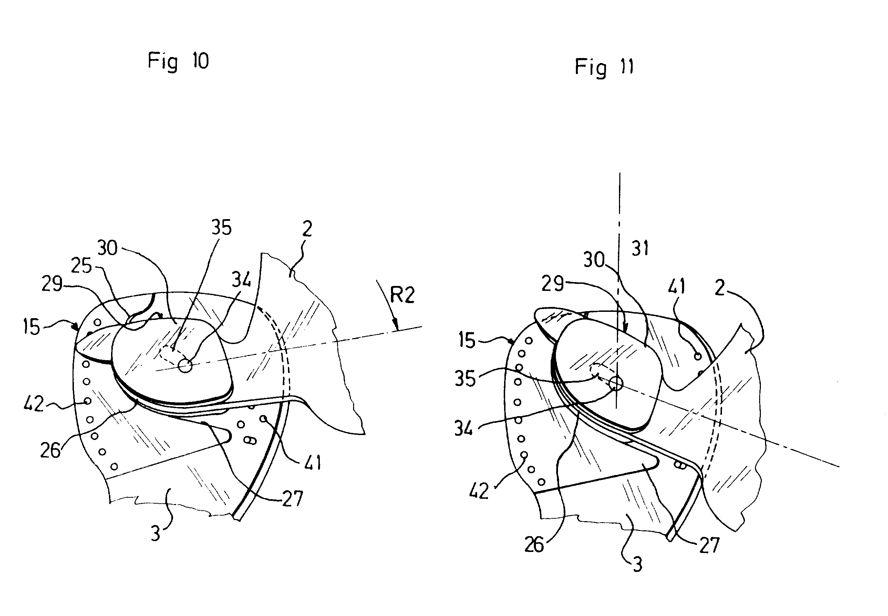

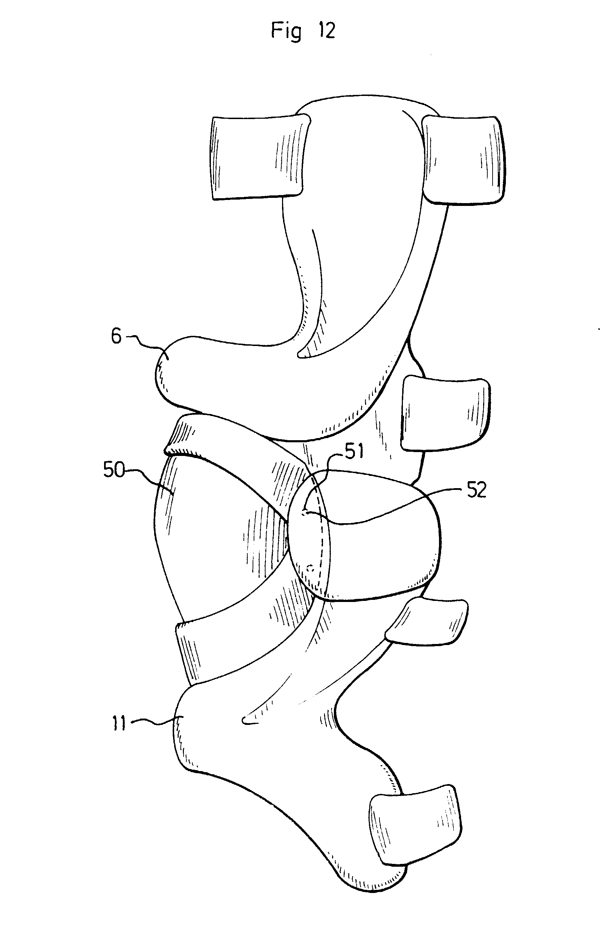

Lorsque la zone de contact arrive à la jonction entre le profil courbe 33 et le profil courbe 33', le doigt 34 vient en butée arrière dans sa lumière oblongue et l'arrondi 32 n'est plus en contact avec la face 25. Une rotation pure R2 est alors obtenue, avec glissement des surfaces de contact, en approximation de la physiologie (la translation devenant faible au-delà de l'angle α) : la face inclinée 27 permet le pivotement jusqu'à la position de flexion complète (figure 11). Les figures 8, 9 et 10 illustrent des positions intermédiaires au cours de la rotation pure R2.

La lumière oblongue 35 contenant le doigt 34 empêche tout déplacement vertical vers le haut de l'armature de fémur et tout effort d'extension qui en résulterait sur les ligaments du genou.

L'articulation proposée permet par ailleurs de limiter autant que nécessaire l'angle de flexion ou d'extension du fémur par rapport au tibia, grâce à la combinaison des logements 41 ou 42 prévus dans les plaques 15 et 16 de l'armature de fémur et du téton 44, lequel, engagé dans un quelconque des logements précédents, sert de butée au bossage 28 qui prolonge vers le bas l'attelle 4.

Les logements 42 et 43, respectivement concernés par l'extension pour les premiers, et par la flexion pour les seconds sont espacés angulairement d'environ 10° de l'un à l'autre, leur nombre étant déterminé de façon à permettre de régler un mouvement de flexion d'amplitude maximale comprise entre 0° et 110° et un mouvement d'extension d'amplitude maximale comprise entre 0° et 90°.

Le boîtier 37 avec son couvercle 38 empêche les tétons 44 de sortir de leurs logements et permet de constituer une réserve pour le ou les tétons 44 que le kinésithérapeute peut à son gré utiliser et placer dans le logement 42 ou 43 approprié pour limiter à la valeur choisie l'angle de flexion ou d'extension autorisé.

La figure 12 schématise une coque de protection de rotule 50 qui peut être fixée sur les deux pièces d'articulation tibiale. En particulier, la plaque interne 16 de chacune de ces articulations est pourvue de deux trous borgnes tels que 51 (figure 1). La coque est munie d'ergots conjugués 52 qui viennent se verrouiller dans ces trous. La coque est en permanence au contact de l'arceau 11 de l'armature tibiale. Elle vient au contact de l'arceau 6 de l'armature fémorale en position d'extension complète.

## Revendications

1. Orthèse de genou, comportant une armature de fémur (2), des moyens (7) de fixation de cette armature de fémur sur la cuisse d'un patient, une armature de tibia (3), des moyens (12) de fixation de cette armature de tibia sur la jambe du patient au-dessous du genou, des moyens (14) d'articulation de l'armature de fémur et de l'armature de tibia comprenant au moins deux pièces d'articulation, dites pièce d'articulation fémorale (29, 30) et pièce d'articulation tibiale (15, 16), respectivement solidaires de l'armature de fémur et de l'armature de tibia et pourvues d'une came d'appui (29, 30) et d'une rampe de guidage (21, 22) agencées pour coopérer en vue d'autoriser et guider le pivotement relatif d'une armature par rapport à l'autre entre une position d'extension complète et une position de flexion complète, des moyens d'arrêt (21, 22, 30, 31; 34, 35) adaptés pour définir la position d'extension complète et interdire une hyperextension, et des moyens d'arrêt (28, 44 ; 34, 35) adaptés pour définir la position de flexion complète et limiter le pivotement à cette position, ladite orthèse étant caractérisée en ce que les moyens d'articulation (14) comprennent :
- de premières surfaces de contact conjuguées (32, 24), portées par la came d'appui (29) et par la rampe de guidage (22) et agencées pour prendre appui et rouler l'une sur l'autre et engendrer, au cours d'une flexion d'angle (α) à partir de la position d'extension complète, une translation vers l'arrière (T) de l'armature de fémur par rapport à l'armature de tibia, se combinant à une rotation (R1) d'une armature par rapport à l'autre,
- un doigt (34) et une lumière oblongue (35) portés par la pièce d'articulation fémorale (4) et par la pièce d'articulation tibiale (15, 16), ledit doigt étant logé dans ladite lumière oblongue, laquelle est agencée pour autoriser le déplacement vers l'arrière dudit doigt au cours de la flexion d'angle (α), et pour arrêter le doigt en position arrière à la fin de ladite flexion d'angle (α) en vue d'interrompre le mouvement de translation vers l'arrière (T) de l'armature de fémur (2) par rapport à l'armature de tibia (3) après le pivotement d'angle (α) à partir de la position d'extension complète,
- de secondes surfaces de contact conjuguées (33, 27) portées par la came d'appui (29) et par la rampe de guidage (22) et agencées pour glisser l'une sur l'autre après la phase de flexion d'angle (α) de façon à engendrer une rotation pure (R2) des armatures autour du doigt (34) arrêté dans sa lumière oblongue (35).

2. Orthèse de genou selon la revendication 1, caractérisée en ce que la came d'appui (29, 30) et la rampe de guidage (21, 22) ainsi que le doigt (34) et la lumière oblongue (35) sont adaptés pour conditionner une translation vers l'arrière de l'armature de fémur par rapport à l'armature de tibia sur un angle de flexion (α) sensiblement compris entre 15° et 25°.

3. Orthèse de genou selon l'une des revendications 1 ou 2, caractérisée en ce que :
- les premières surfaces de contact comprennent, sur la came d'appui (29), un profil arrondi (32) de faible rayon de courbure, prolongé par un profil courbe (33), et sur la rampe de guidage (22), une encoche ouverte (24) prolongée des deux côtés de faces planes (26, 25), le profil arrondi (32) venant se loger dans l'encoche ouverte (24) pendant la flexion d'angle (α) pour servir de guide de roulement, avec un appui sur la face (25) servant de butée avant, cependant que le profil courbe (33) vient rouler sur la face plane (26),
- les secondes surfaces de contact comprennent, sur la came d'appui un profil courbe (33'), et sur la rampe de guidage, une face inclinée (27), ledit profil courbe (33') venant glisser sur ladite face inclinée (27) après la phase de flexion d'angle (α).

4. Orthèse de genou selon l'une des revendications 1, 2 ou 3, caractérisée en ce que :
- la pièce d'articulation fémorale comprend deux plaques identiques (29, 30) fixées de part et d'autre pour former la came d'appui,
- la pièce d'articulation tibiale comprend deux plaques espacées (15, 16) sur lesquelles sont fixées des guides plats (21, 22) pour former la rampe de guidage.

5. Orthèse de genou selon la revendication 4, caractérisée en ce que le doigt (34) est monté dans un trou (36) de la pièce d'articulation fémorale pour traverser de part en part celle-ci et venir se loger dans des lumières oblongues (35) ménagées en correspondance dans les plaques espacées (15, 16) de la pièce d'articulation tibiale.

6. Orthèse de genou selon l'une des revendications 1, 2 ou 3, ou 4, ou 5, comprenant des moyens réglables de limitation de la flexion et de l'extension, comportant une pluralité de logements (42, 43) répartis sur la pièce d'articulation tibiale (15, 16), un bossage (28) solidaire de la pièce d'articulation fémorale (4) et au moins un téton (44) logé dans un desdits logements en vue de servir de butée au bossage (28) dans la position limite désirée de flexion ou d'extension.

7. Orthèse de genou selon la revendication 6, caractérisée en ce que les logements (42, 43) recevant le téton (44) sont répartis sur un arc de courbe et espacés avec un écartement angulaire déterminé.

8. Orthèse de genou selon la revendication 7, caractérisée en ce que l'écartement angulaire est sensiblement égal d'un logement au suivant et correspond sensiblement à un angle de 10°, le nombre des logements étant choisi de façon à permettre de régler un mouvement de flexion d'amplitude maximale comprise entre 0° et 110° et un mouvement d'extension d'amplitude maximale comprise entre 0° et 90°.

9. Orthèse de genou selon les revendications 4 et 6 prises ensemble, caractérisée en ce que la plaque (15) de la pièce d'articulation tibiale, située vers l'extérieur de l'articulation, comporte un couvercle (38), délimitant avec cette plaque un espace fermé formant boîtier (37), propre à contenir un ou plusieurs tétons de butée (44).

10. Orthèse de genou selon la revendication 8, caractérisée en ce que le boîtier (37) comporte une ouverture interne (39) en regard de la lumière oblongue (35), le doigt (34) comportant une tête épaulée (40) disposée de l'autre côté du fond (41) du boîtier (37), de manière à rendre ce doigt prisonnier du boîtier sans empêcher son déplacement dans la lumière oblongue.

11. Orthèse de genou selon l'une des revendications 1 à 10, caractérisée en ce qu'elle comprend deux pièces d'articulation fémorale et deux pièces d'articulation tibiale, destinées à coopérer par couples de part et d'autre du genou, une coque de protection de rotule (50) étant fixée sur les deux pièces d'articulation tibiale.

12. Orthèse de genou selon l'une des revendications 1 à 11, caractérisée en ce que l'armature de fémur (2) et l'armature de tibia (3) comprennent chacune deux attelles latérales rigides (4, 5 ; 9, 10) réunies par un arceau antérieur (6, 11), l'arceau antérieur (6) reliant les attelles (4, 5) de l'armature de fémur étant disposé à la base de celle-ci en vue de procurer un appui au bas de la cuisse du patient à proximité immédiate du genou.
